# EUROPÄISCHE PATENTSCHRIFT

(11) **EP 2 490 993 B1**
(45) Veröffentlichungstag und Bekanntmachung des Hinweises auf die Patenterteilung: **11.06.2014**
(21) Anmeldenummer: 10768923.4
(22) Anmeldetag: 19.10.2010
(51) Int. Cl.: C07C 29/56, C07C 33/20, C07C 45/29, C07C 47/228, C11B 9/00

(54) **VERFAHREN ZUR HERSTELLUNG M-SUBSTITUIERTER PHENYLALKANOLE DURCH ISOMERISIERUNG**
METHOD FOR PRODUCING M-SUBSTITUTED PHENYLALKANOLS BY MEANS OF ISOMERIZATION
PROCÉDÉ DE PRODUCTION DE PHÉNYLALCANOLS À SUBSTITUTION M PAR ISOMÉRISATION

(30) Priorität: 23.10.2009 EP 09173907
(43) Veröffentlichungstag der Anmeldung: 29.08.2012
(73) Patentinhaber: BASF SE, 67056 Ludwigshafen (DE)
(72) Erfinder: LANVER, Andreas, 68165 Mannheim (DE); EBEL, Klaus, 68623 Lampertheim (DE); BECK, Karl, 76684 Östringen (DE); PELZER, Ralf, 37699 Fürstenberg (DE); BOTZEM, Jörg, 67117 Limburgerhof (DE); GRIESBACH, Ulrich, 68305 Mannheim (DE)
(86) Internationale Anmeldenummer: PCT/EP2010/065673
(87) Internationale Veröffentlichungsnummer: WO 2011/048068

(56) Entgegenhaltungen:
- EP-A1- 0 045 571
- JP-A- 2 009 830
- JP-A- 2 238 097
- Innospec: "Mefloral", , 2008, XP002620576, Gefunden im Internet: URL:http://www.innospecinc.com/assets/_fil es/documents/apr_08/cm__1209463592_Meflora l-Green.pdf [gefunden am 2011-02-04]

## Beschreibung

Gegenstand der vorliegenden Erfindung ist ein Verfahren zur Herstellung m-substituierter Phenylalkanole durch Isomerisierung p-substituierter Phenylalkanole. Die m-substituierten Phenylalkanole sowie die aus diesen hergestellten m-substituierten Phenylalkanale, beispielsweise Derivate des Geruchstoffs 3-Phenyl-1-propanol, sind als Aromachemikalien von Interesse.

Zur Herstellung m-substituierter Phenylalkanole sind verschiedene Synthesen bekannt. In der WO 2008/053148 ist eine 3-stufige Synthese zur Herstellung von 3-(3-tert-Butyl-phenyl)propanal ausgehend von 1-tert-Butyl-3-ethylbenzol beschrieben. Dabei wird die Ausgangsverbindung zunächst zum 1-tert-Butyl-3-(1-brom-ethyl)benzol bromiert und anschließend zu dem entsprechenden substituierten Styrol eliminiert. Durch Hydrofor-mylierung erhält man anschließend das 3-(3-tert-Butylphenyl)propanal. Für einen grosstechnischen Prozess erscheint diese Synthese aufgrund geringer Ausbeuten weniger geeignet.

Die Herstellung von 2-Methyl-3-(3-tert-butylphenyl)propanal und von 2-Methyl-3-(3-isobutylphenyl)propanal gelingt Ishii et al. (J. Org. Chem. 2005, 70, 5471-5474) durch Palladium-Katalysierte oxidative Kupplung von tert-Butylbenzol bzw. Isopropylbenzol mit Methacrolein gefolgt von einer Palladium-Katalysierten Hydrierung. Bei dem Kupplungsschritt wird ein Katalysatorsystem bestehend aus Pd(OAc)₂ und H₄PMo₁₁VO₄₀ x 26 H2O eingesetzt. Es wird eine hohe Katalysatormenge von ca. 7 mol% benötig. Bei einer Ausbeute von ca. 65% beträgt das m/p-Verhältnis 56/44 (für 2-Methyl-3-(3-tertbutylphenyl)propanal) bzw. 51/40 (für 2-Methyl-3-(3-iso-butylphenyl)propanal). Auch dieses Verfahren erscheint für einen grosstechnischen Prozess weniger geeignet, da die Menge des teuren eingesetzten Katalysators hoch ist und die Selektivität zu dem m-Isomeren gering ist.

In der EP 0 045 571 ist die Friedel-Crafts Alkylierung von 2-Methyl-3-phenylpropanol zu 2-Methyl-3-(3-tert-butylphenyl)propanol und 2-Methyl-3-(4-tert-butylphenyl)propanol beschrieben. Als Alkylierungsreagenzien werden Isobutylen, Diisobutylen und tert-Butylchlorid eingesetzt. Als Katalysatoren werden Eisenchlorid und Phosphorsäure und als Lösungsmittel Methylenchlorid oder Phosphorsäure verwendet. In Abhängigkeit der Reaktionsbedingungen und des Katalysators werden m/p-Verhältnisse von 1/13 bis 1/5 erhalten. Die Gesamtausbeuten (m-Isomer und p-Isomer) betragen bis zu 52%.

In der DE 29 52 719 wird ebenfalls die Eisenchlorid-Katalysierte Friedel-Crafts Alkylierung von 2-Methyl-3-phenylpropanol beschrieben. In Cyclohexan oder Dichlorethan als Lösungsmittel wurde eine Ausbeute von 84-86% an 2-Methyl-3-(4-tert-butylphenyl)-propanol erhalten. Die Bildung der m-isomeren Verbindung, (2-Methyl-3-(3-tert-butylphenyl)-propanol), wurde nicht nachgewiesen. Nachteil der beschrieben Friedel-Crafts Alkylierungen ist die geringe Menge der gebildeten m-isomeren Verbindung (Verhältnis m : p ist max. 1 : 5).

Demgegenüber ermöglicht das erfindungsgemässe Verfahren, dass man die m-substituierten Phenylalkanole, die als Vorstufe für die sehr interessanten entsprechend substituierten Phenylalkanale dienen, kostengünstig und in guter Ausbeute auf einfachste Weise herstellt.

Die vorliegende Erfindung betrifft ein Verfahren zur Herstellung von in m-Stellung substituierten Phenylalkanolen, welche durch Isomerisierung von in p-Stellung substituierten Phenylalkanolen erhalten werden können. Die Isomerisierung erfolgt an bestimmten Friedel-Crafts Katalysatoren. Gegenstand der Erfindung ist somit ein Verfahren zur Herstellung m-substituierter Phenylalkanole der Formel (I) 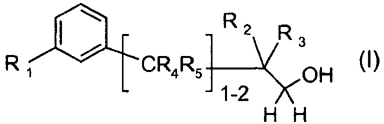 worin R₁ C₁-C₅-Alkyl ist und R₂, R₃, R₄ und R₅ unabhängig voneinander Wasserstoff oder Methyl bedeuten, welches dadurch gekennzeichnet ist, dass man ein p-substituiertes Phenylalkanol der Formel (II) 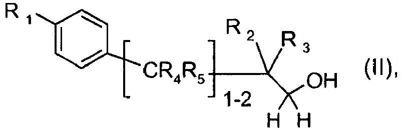 worin R₁, R₂, R₃, R₄ und R₅ die unter Formel (I) angegebenen Bedeutungen haben, in Gegenwart eines Friedel-Crafts Katalysators zu einem m-substituierten Phenylalkanol der Formel (I) isomerisiert.

Es wurde überraschend gefunden, dass bei der Isomerisierungsreaktion von p-substituierten Phenylalkanolen zu m-substituierten Phenylalkanolen unter bestimmten Bedingungen m/p-Isomerenverhältnisse von > 1/1 erhalten werden können.

Als Alkylreste R₁ kommen z.B. in Betracht: Methyl, Ethyl, n-Propyl, Isopropyl, n-Butyl, Isobutyl, sec-Butyl, tert-Butyl, n-Pentyl. Bevorzugte Alkylreste R₁ sind: Ethyl, Isopropyl, Isobutyl und tert-Butyl.

Bevorzugt ist ein Verfahren zur Herstellung m-substituierter Phenylpropanole der Formel (III) 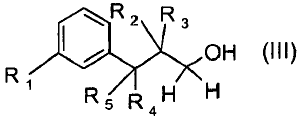 worin R₁, R₂, R₃, R₄ und R₅ die unter Formel (I) angegebenen Bedeutungen haben, das dadurch gekennzeichnet ist, dass man ein p-substituiertes Phenylpropanol der Formel (IV) 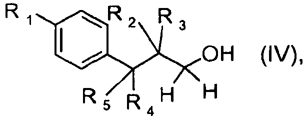 worin R₁, R₂, R₃, R₄ und R₅ die unter Formel (I) angegebenen Bedeutungen haben, in Gegenwart eines Friedel-Crafts Katalysators zu einem m-substituierten Phenylalkanol der Formel (III) isomerisiert. Vorzugsweise bedeutet R₁ Ethyl, Isopropyl, Isobutyl oder tert-Butyl.

Besonders bevorzugt ist ein Verfahren welches dadurch gekennzeichnet ist, dass man von einem p-substituierten Phenylpropanol der Formel (V) ausgeht 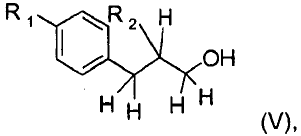 worin R1 und R2 die unter Formel (I) angegebenen Bedeutungen haben. Vorzugsweise bedeutet R1 Ethyl, Isopropyl, Isobutyl oder tert-Butyl.

Ein ganz besonders bevorzugtes Verfahren ist dadurch gekennzeichnet, dass man als Ausgangsverbindung 2-Methyl-3-(4-tert.-butylphenyl)propanol in Gegenwart eines Friedel-Crafts Katalysators, insbesondere in Gegenwart von Aluminiumtrichlorid (AlCl₃), zu 2-Methyl-3-(3-tert.-butylphenyl)propanol isomerisiert.

Ein ebenfalls besonders bevorzugtes Verfahren ist dadurch gekennzeichnet, dass man als Ausgangverbindung 2-Methyl-3-(4-iso-butylphenyl)propanol, 3-(4-tert-Butyl-phenyl)propanol, 2-Methyl-3-(4-isopropylphenyl)propanol, 3-(4-Ethylphenyl)-2,2-dimethylpropanol oder 3-(4-Isopropylphenyl)butanol einsetzt und die Isomerisierung in Gegenwart eines Friedel-Crafts-Katalysators zu den Verbindungen 2-Methyl-3-(3-isobutylphenyl)propanol, 3-(3-tert-Butylphenyl)propanol, 2-Methyl-3-(3-isopropyl-phenyl)propanol, 3-(3-Ethylphenyl)-2,2-dimethylpropanol oder 3-(3-Isopropylphenyl)butanol durchführt.

Als Katalysatoren können typische Friedel-Crafts Katalysatoren eingesetzt werden. Als Beispiele seien genannt AlCl₃, AlBr₃, TiCl₄, ZrCl₄, VCl₃, ZnCl₂, FeBr₃ und FeCl₃. Bevorzugt werden die Friedel-Crafts Katalysatoren AlCl₃ oder AlBr₃ verwendet. Es werden im allgemeinen Katalysatormengen von 1 bis 200 mol% bezogen auf die Molmenge der eingesetzten p-substituierten Phenylalkanolverbindung eingesetzt. Bevorzugt sind Katalysatormengen von 33% bis 110 mol% bezogen auf die Molmenge der eingesetzten p-substituierten Phenylalkanolverbindung.

Die Isomerisierung erfolgt bei Temperaturen zwischen 0°C und 100°C. Besonders bevorzugt sind Temperaturen zwischen 10°C und 50°C. Die Reaktionszeiten betragen 30 Minuten bis 24 Stunden. Besonders bevorzugt sind Reaktionszeiten zwischen 1 Stunde und 6 Stunden.

Die Reaktion kann lösungsmittelfrei oder in einem Lösungsmittel durchgeführt werden. Geeignete Lösungsmittel sind: Cyclohexan, Toluol, p-tert-Butyltoluol, Dichlormethan, 1,2-Dichlorethan, Chlorbenzol, Dichlorbenzol. Besonders bevorzugt sind Dichlormethan und Chlorbenzol.

Bevorzugte Ausgangsstoffe für die Isomerisierung sind die folgenden Substrate: 2-Methyl-3-(4-tert-butylphenyl)propanol, 2-Methyl-3-(4-iso-butylphenyl)propanol, 3-(4-tert-Butylphenyl)propanol, 2-Methyl-3-(4-isopropylphenyl)propanol, 3-(4-Ethylphenyl)-2,2-dimethylpropanol, 3-(4-Isopropylphenyl)butanol. Daraus ergeben sich die folgenden m-Isomere als Hauptprodukte (in einer Ausbeute von >25%, mit einem Isomerenverhältnis m/p > 1/1) der Reaktion: 2-Methyl-3-(3-tert-butylphenyl)propanol, 2-Methyl-3-(3-iso-butylphenyl)propanol, 3-(3-tert-Butylphenyl)propanol, 2-Methyl-3-(3-isopropylphenyl)propanol und 3-(3-Ethylphenyl)-2,2-dimethylpropanol, 3-(3-Isopropylphenyl)butanol. Besonders bevorzugt ist das Substrat 2-Methyl-3-(4-tertbutylphenyl)propanol.

Die Reaktion wird in der Regel so durchgeführt, dass der Katalysator in das im Lösungsmittel gelöste Phenylalkanol eingetragen wird. Die Aufarbeitung erfolgt durch Aufarbeitung mit Wasser, vorzugsweise in Gegenwart von Alkali, so dass ein alkalischer pH-Wert eingestellt wird, ganz besonders bevorzugt in Gegenwart von Alkalilauge, wie z.B. Natronlauge und/oder Kalilauge, sowie durch Destillation des Lösungsmittels. Die Aufreinigung des Rohproduktes und die Isolierung der m-substituierten Phenylalkanole erfolgt in der Regel durch Destillation.

Die bei der Reaktion entstehenden am Aromaten mono- und tri-substituierten Phenylalkanole der Formeln 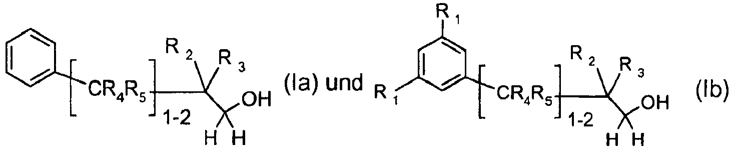 worin R₁, R₂, R₃, R₄ und R₅ die unter Formel (I) angegebenen Bedeutungen haben, sind Nebenprodukte, die der Isomerisierungsreaktion wieder zugeführt werden können. Durch die Rückführung dieser Produkte in die Reaktionsmasse wird das Gleichgewicht zwischen der p-substituierten und der m-substituierten Komponente und den mono-und tri-substituierten Phenylalkanolen immer wieder neu eingestellt, wodurch ein erhöhter Anteil an dem gewünschten m-substituierten Produkt erhalten wird, da dieses der Reaktionsmasse vor jeder Rückführung (nach Aufarbeitung und Destillation) entzogen wird.

Die erfindungsgemäss hergestellten Alkanole der Formel (I) können basierend auf an sich bekannten Dehydrierungs- oder Oxidationsmethoden in die entsprechenden Aldehyde überführt werden (vergl. z.B.: Houben-Weyl" Methoden der organischen Chemie" , Band 7/1, S. 160ff, S. 171f). Besonders interessante Verbindungen dieser Substanzklasse sind 2-Methyl-3-(3-tert-butylphenyl)propanal, 2-Methyl-3-(3-iso-butylphenyl)propanal, 3-(3-tert-Butylphenyl)propanal, 2-Methyl-3-(3-isopropylphenyl)-propanal, 3-(3-Ethylphenyl)-2,2-dimethylpropanal und 3-(3-Isopropylphenyl)butanal.

Wie in der EP-A-0 045 571 beschrieben können Phenylpropanole durch Oxidation oder Dehydrierung in die entsprechenden Phenylpropanale überführt werden. Diese Umsetzung gelingt beispielsweise durch Kupferchromit-katalysierte Flüssigphasendehydrierung.

Bevorzugte Edukte dieser Umsetzung zum Aldehyd sind 2-Methyl-3-(3-tert-butyl-phenyl)-propanol, 2-Methyl-3-(3-iso-butylphenyl)propanol, 3-(3-tert-Butylphenyl)-propanol, 2-Methyl-3-(3-isopropylphenyl)propanol, 3-(3-Ethylphenyl)-2,2-dimethyl-propanol, 3-(3-Isopropylphenyl)butanol. Daraus ergeben sich die folgenden Aldehyde: 2-Methyl-3-(3-tert-butylphenyl)propanal, 2-Methyl-3-(3-iso-butylphenyl)propanal, 3-(3-tert-Butylphenyl)propanal, 2-Methyl-3-(3-isopropylphenyl)propanal, 3-(3-Ethylphenyl)-2,2-dimethylpropanal, 3-(3-Isopropylphenyl)butanal.

Ein weiterer Gegenstand der Erfindung ist somit die Herstellung der aus den m-substituierten Phenylalkanolen der Formel (I) durch Oxidation oder Dehydrierung erhältlichen Wertprodukte der Formel (VI), die als Duftstoffe und Aromachemikalien bekannt sind, 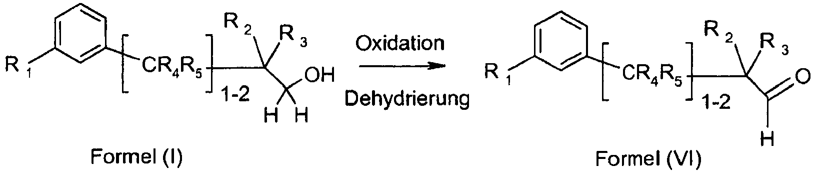 worin R₁, R₂, R₃, R₄ und R₅ die unter Formel (I) angegebenen Bedeutungen haben. Das erfindungsgemässe Verfahren zur Herstellung von Duftstoffen der Formel (VI) 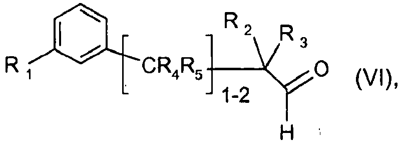 worin R₁ C₁-C₅-Alkyl ist und R₂, R₃, R₄ und R₅ unabhängig voneinander Wasserstoff oder Methyl bedeuten, ist dadurch gekennzeichnet, dass man ein p-substituiertes Phenylalkanol der Formel (II) 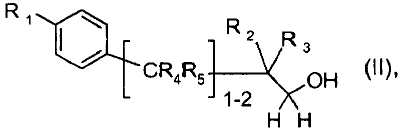 worin R₁, R₂, R₃, R₄ und R₅ die unter Formel (I) angegebenen Bedeutungen haben, in Gegenwart eines Friedel-Crafts Katalysators zu einem m-substituierten Phenylalkanol der Formel (I) 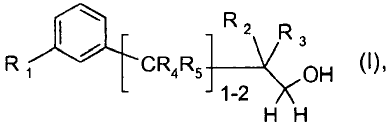 worin R₁, R₂, R₃, R₄ und R₅ die unter Formel (I) angegebenen Bedeutungen haben, isomerisiert, und anschliessend das erhaltene m-substituierte Phenylalkanol der Formel (I) durch Oxidation oder Dehydrierung in das m-substituierte Phenylalkanal der Formel (VI) überführt.

Die gemäss dem Verfahren erhaltenen Aldehyde der Formel (VI) sind zum Teil bekannt und teilweise neue Duft- und Aromastoffe. Ein neuer Duft- und Aromastoff entspricht der Formel (VII)

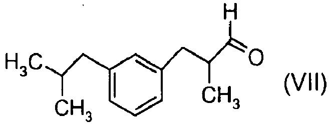

Es wurde ferner überraschenderweise gefunden, dass sich durch Kombination eines oder mehrerer der gemäss dem erfindungsgemässen Verfahren erhaltenen Aldehyde der Formel (VI) zusammen mit anderen Aromachemikalien toxikologisch vorteilhafte Formulierungen herstellen lassen.

Die Erfindung wird durch die folgenden Beispiele näher veranschaulicht. In den Beispielen verstehen sich alle Angaben in % als mol.-%.

### Beispiele

### Beispiel 1

327g (1,50 mol) 2-Methyl-3-(4-tert-butylphenyl)propanol wurden in 500g Dichlormethan vorgelegt. Innerhalb von 4h wurden 220g (1,65 mol) AlCl₃ portionsweise bei Raumtemperatur zugegeben. Es wurde weitere 60 Minuten bei Raumtemperatur gerührt. Zur Aufarbeitung wurde das Reaktionsgemisch langsam in 1050g Eiswasser gegeben. Es wurde auf Raumtemperatur erwärmt und 670g 50%ige Natronlauge zugegeben. Die Phasen wurden getrennt und die organische Phase mit Wasser (2 x 200ml) gewaschen. Das Lösungsmittel wurde abdestilliert und das Rohprodukt bei 1 mbar fraktioniert destilliert. Es wurden 104g 2-Methyl-3-(3-tert-butylphenyl)propanol (33%), 81g 2-Methyl-3-(4-tert-butylphenyl)propanol (26%) erhalten. Als Nebenprodukte wurden 2-Methyl-3-Phenylpropanol und 3-(3,5-Di-tert-Butylphenyl)-2-methyl-propanol erhalten (zusammen ca. 35 %).

### Beispiel 2

2,1g eines Gemisches bestehend aus 2-Methyl-3-(3-tert-butylphenyl)propanol (30%), 2-Methyl-3-(4-tert-butylphenyl)propanol (48%) und 3-(3,5-Di-tert-Butylphenyl)-2-methyl-propanol (18%) wurden mit 2;1g 2-Methyl-3-Phenylpropanol und 6,3g Dichlormethan versetzt. 2,9g AlCl₃ wurden portionsweise bei Raumtemperatur zugegeben. Nachdem 7 Stunden bei Raumtemperatur gerührt wurde, wurde mit Wasser und Natronlauge aufgearbeitet und das Lösungsmittel entfernt. Es wurde ein Gemisch mit der folgenden Zusammensetzung erhalten: 2-Methyl-3-(3-tert-butylphenyl)propanol (34%); 2-Methyl-3-(4-tert-butylphenyl)propanol (21 %); 3-(3,5-Di-tert-Butylphenyl)-2-methyl-propanol (5%); 2-Methyl-3-Phenylpropanol (37%).

### Beispiel 3

2,1g eines Gemisches bestehend aus 2-Methyl-3-(3-tert-butylphenyl)propanol (30%), 2-Methyl-3-(4-tert-butylphenyl)propanol (48%) und 3-(3,5-Di-tert-Butylphenyl)-2-methyl-propanol (18%) wurden mit 2,1g 2-Methyl-3-Phenylpropanol und 6,5g 4-tert-Butyltoluol versetzt. 2,9g AlCl₃ wurden portionsweise bei Raumtemperatur zugegeben. Nachdem 7 Stunden bei Raumtemperatur gerührt wurde, wurde mit Wasser und Nat-ronlauge aufgearbeitet und das Lösungsmittel entfernt. Es wurde ein Gemisch mit der folgenden Zusammensetzung erhalten: 2-Methyl-3-(3-tert-butylphenyl)propanol (38%); 2-Methyl-3-(4-tert-butylphenyl)propanol (23%); 3-(3,5-Di-tert-Butylphenyl)-2-methyl-propanol (17%); 2-Methyl-3-Phenylpropanol (12%).

### Beispiel 4

2,1 g eines Gemisches bestehend aus 2-Methyl-3-(3-tert-butylphenyl)propanol (30%), 2-Methyl-3-(4-tert-butylphenyl)propanol (48%) und 3-(3,5-Di-tert-Butylphenyl)-2-methyl-propanol (18%) wurden mit 2,1g 2-Methyl-3-Phenylpropanol versetzt. 2,9g AlCl3 wurden portionsweise bei Raumtemperatur zugegeben. Nachdem 7 Stunden bei Raumtemperatur gerührt wurde, wurde mit Wasser und Natronlauge aufgearbeitet. Es wurde ein Gemisch mit der folgenden Zusammensetzung erhalten: 2-Methyl-3-(3-tert-butylphenyl)propanol (31 %); 2-Methyl-3-(4-tert-butylphenyl)propanol (17%); 3-(3,5-Di-tert-Butylphenyl)-2-methyl-propanol (5%); 2-Methyl-3-Phenylpropanol (32%).

## Patentansprüche

1. Verfahren zur Herstellung m-substituierter Phenylalkanole der Formel (I) 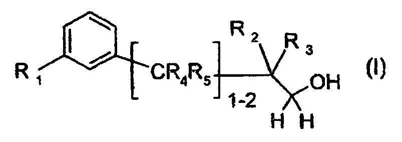 worin R₁ C₁-C₅-Alkyl ist und R₂, R₃, R₄ und R₅ unabhängig voneinander Wasserstoff oder Methyl bedeuten, **dadurch gekennzeichnet, dass** man ein p-substituiertes Phenylalkanol der Formel (II) 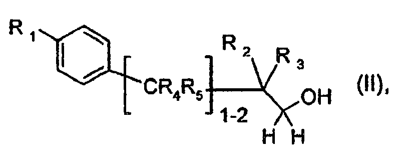 worin R₁, R₂, R₃, R₄ und R₅ die unter Formel (I) angegebenen Bedeutungen haben, in Gegenwart eines Friedel-Crafts Katalysators zu einem m-substituierten Phenylal-kanol der Formel (I) isomerisiert.

2. Verfahren gemäss Anspruch 1 zur Herstellung m-substituierter Phenylpropanole der Formel (III) 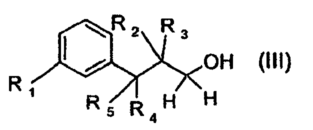 worin R₁, R₂, R₃, R₄ und R₅ die in Anspruch 1 angegebenen Bedeutungen haben, **dadurch gekennzeichnet, dass** man ein p-substituiertes Phenylpropanol der Formel (IV) 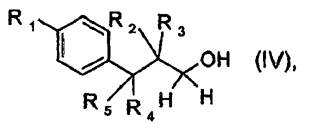 worin R₁, R₂, R₃, R₄ und R₅ die in Anspruch 1 angegebenen Bedeutungen haben, in Gegenwart eines Friedel-Crafts Katalysators zu einem m-substituierten Phenylalkanol der Formel (III) isomerisiert.

3. Verfahren gemäss einem der Ansprüche 1 und 2, **dadurch gekennzeichnet, dass** man von einem p-substituierten Phenylpropanol der Formel (V) ausgeht 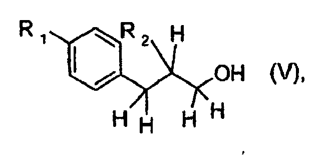 worin R₁ und R₂ die in Anspruch 1 angegebenen Bedeutungen haben.

4. Verfahren gemäss einem der Ansprüche 1 bis 3, **dadurch gekennzeichnet, dass** man von einem p-substituierten Phenylpropanol ausgeht, worin R₁ Ethyl, Isopropyl, Isobutyl und tert-Butyl ist

5. Verfahren gemäss einem der Ansprüche 1 bis 4, **dadurch gekennzeichnet, dass** man als Friedel-Crafts Katalysator AlCl₃, AlBr₃, TiCl₄, ZrCl₄, VCl₃, ZnCl₂, FeBr₃, FeCl₃ verwendet.

6. Verfahren gemäss Anspruch 5, **dadurch gekennzeichnet, dass** man als Friedel-Crafts Katalysator AlCl₃ oder AlBr₃ verwendet.

7. Verfahren gemäss einem der Ansprüche 1 bis 6, **dadurch gekennzeichnet, dass** man den Friedel-Crafts Katalysator in einer Menge von 1 bis 200 mol.-%, insbesondere zwischen 33 und 110 mol.-%, bezogen auf die Molmenge des eingesetzten p-substituierten Phenylpropanols, einsetzt.

8. Verfahren gemäss einem der Ansprüche 1 bis 7, **dadurch gekennzeichnet, dass** man die Isomerisierung bei einer Temperatur zwischen 0 und 100°C durchführt.

9. Verfahren gemäss einem der Ansprüche 1 bis 7, **dadurch gekennzeichnet, dass** man 2-Methyl-3-(4-tert.-butylphenyl)propanol zu 2-Methyl-3-(3-tert.-butylphenyl)propanol isomerisiert.

10. Verfahren gemäss einem der Ansprüche 1 bis 7, **dadurch gekennzeichnet, dass** man ausgehend von 2-Methyl-3-(4-iso-butylphenyl)propanol, 3-(4-tert-Butyl-phenyl)propanol, 2-Methyl-3-(4-isopropylphenyl)propanol, 3-(4-Ethylphenyl)-2,2-dimethylpropanol oder 3-(4-Isopropylphenyl)butanol die Isomerisierung durchführt.

11. Verfahren zur Herstellung von Duftstoffen der Formel (VI) 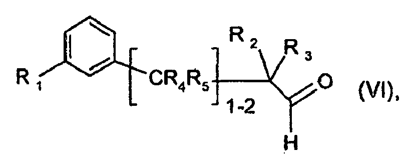 worin R₁ C₁-C₅-Alkyl ist und R₂, R₃, R₄ und R₅ unabhängig voneinander Wasserstoff oder Methyl bedeuten, **dadurch gekennzeichnet, dass** man ein m-substituiertes Phenylalkanol der Formel (I) 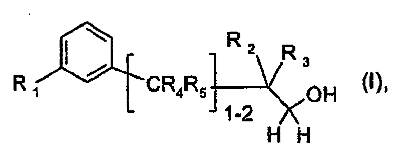 nach dem Verfahren gemäß Anspruch 1 herstellt und anschliessend das erhaltene m-substituierte Phenylalkanol der Formel (I) durch Oxidation oder Dehydrierung in das m-substituierte Phenylalkanal der Formel (VI) überführt.

## Claims

1. A process for the preparation of m-substituted phenylalkanols of the formula (I) 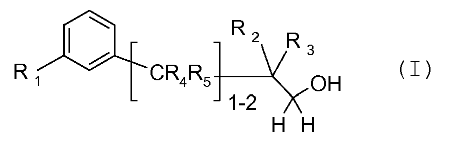 in which R₁ is C₁-C₅-alkyl and R₂, R₃, R₄ and R₅, independently of one another, are hydrogen or methyl, wherein a p-substituted phenylalkanol of the formula (II) 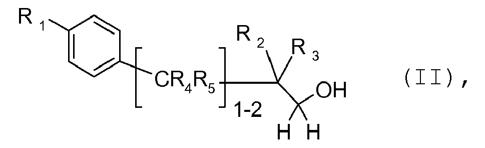 in which R₁, R₂, R₃, R₄ and R₅ have the meanings given under formula (I), is isomerized in the presence of a Friedel-Crafts catalyst to give an m-substituted phenylalkanol of the formula (I).

2. The process according to claim 1 for the preparation of m-substituted phenylpropanols of the formula (III) 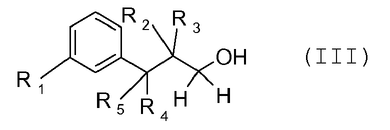 in which R₁, R₂, R₃, R₄ and R₅ have the meanings given in claim 1, wherein a p-substituted phenylpropanol of the formula (IV) 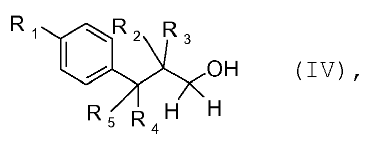 in which R₁, R₂, R₃, R₄ and R₅ have the meanings given in claim 1, is isomerized in the presence of a Friedel-Crafts catalyst to give an m-substituted phenylalkanol of the formula (III).

3. The process according to either of claims 1 and 2, wherein the starting compound used is a p-substituted phenylpropanol of the formula (V) 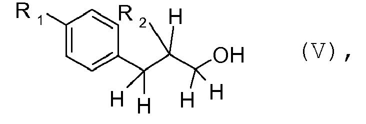 in which R₁ and R₂ have the meanings given in claim 1.

4. The process according to any one of claims 1 to 3, wherein the starting compound is a p-substituted phenylpropanol in which R₁ is ethyl, isopropyl, isobutyl and tert-butyl.

5. The process according to any one of claims 1 to 4, wherein the Friedel-Crafts catalyst used is AlCl₃, AlBr₃, TiCl₄, ZrCl₄, VCl₃, ZnCl₂, FeBr₃, FeCl₃.

6. The process according to claim 5, wherein the Friedel-Crafts catalyst used is AlCl₃ or AlBr₃.

7. The process according to any one of claims 1 to 6, wherein the Friedel-Crafts catalyst is used in an amount of from 1 to 200 mol%, in particular between 33 and 110 mol%, based on the molar amount of the p-substituted phenylpropanol used.

8. The process according to any one of claims 1 to 7, wherein the isomerization is carried out at a temperature between 0 and 100°C.

9. The process according to any one of claims 1 to 7, wherein 2-methyl-3-(4-tert-butylphenyl)propanol is isomerized to give 2-methyl-3-(3-tert-butylphenyl)propanol.

10. The process according to any one of claims 1 to 7, wherein the isomerization is carried out starting from 2-methyl-3-(4-isobutylphenyl)propanol, 3-(4-tert-butylphenyl)propanol, 2-methyl-3-(4-isopropylphenyl)propanol, 3-(4-ethylphenyl)-2,2-dimethylpropanol or 3-(4-isopropylphenyl)butanol.

11. A process for the preparation of fragrances of the formula (VI) 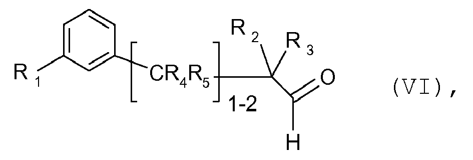 in which R₁ is C₁-C₅-alkyl and R₂, R₃, R₄ and R₅, independently of one another, are hydrogen or methyl, wherein an m-substituted phenylalkanol of the formula (I) 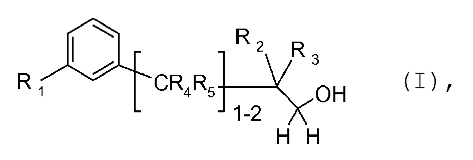 is produced by the process according to claim 1, and then the resulting m-substituted phenylalkanol of the formula (I) is converted to the m-substituted phenylalkanal of the formula (VI) by oxidation or dehydrogenation.

## Revendications

1. Procédé pour la préparation de phénylalcanols m-substitués de formule (I) 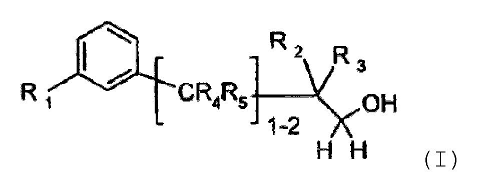 dans laquelle R₁ est un groupe alkyle en C₁-C₅ et R₂, R₃, R₄ et R₅ représentent, indépendamment les uns des autres, un atome d'hydrogène ou le groupe méthyle, **caractérisé en ce qu'**on isomérise un phénylalcanol p-substitué de formule (II) 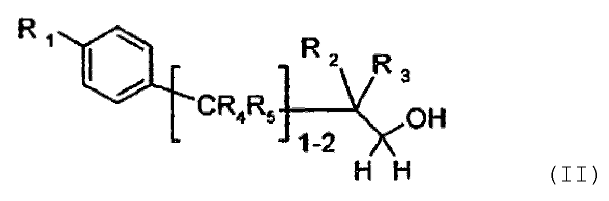 dans laquelle R₁, R₂, R₃, R₄ et R₅ ont les significations indiquées au-dessous de la formule (I), en présence d'un catalyseur de Friedel-Crafts, pour aboutir à un phénylalcanol m-substitué de formule (I).

2. Procédé selon la revendication 1 pour la préparation de phénylpropanols m-substitués de formule (III) 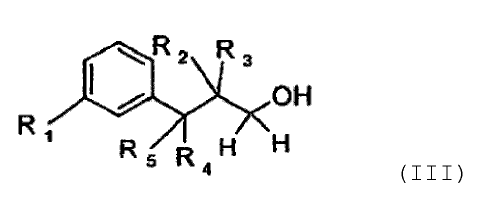 dans laquelle R₁, R₂, R₃, R₄ et R₅ ont les significations indiquées dans la revendication 1, **caractérisé en ce qu'**on isomérise un phénylpropanol p-substitué de formule (IV) 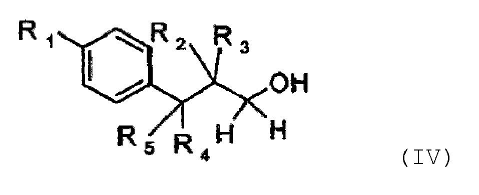 dans laquelle R₁, R₂, R₃, R₄ et R₅ ont les significations indiquées dans la revendication 1, en présence d'un catalyseur de Friedel-Crafts, pour obtenir un phénylalcanol m-substitué de formule (III).

3. Procédé selon l'une quelconque des revendications 1 et 2, **caractérisé en ce qu'**on part d'un phénylpropanol p-substitué de formule (V) 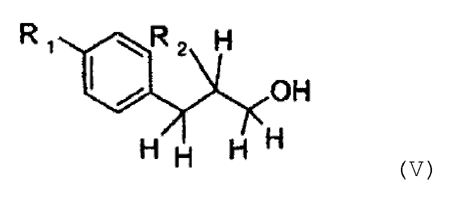 dans laquelle R₁ et R₂ ont les significations indiquée dans la revendication 1

4. Procédé selon l'une quelconque des revendications 1 à 3, **caractérisé en ce qu'**on part d'un phénylpropanol p-substitué, dans lequel R₁ est le groupe éthyle, isopropyle, isobutyle et tert-butyle.

5. Procédé selon l'une quelconque des revendications 1 à 4, **caractérisé en ce qu'**on utilise comme catalyseur de Friedel-Crafts AlCl₃, AlBr₃, TiCl₄, ZrCl₄, VCl₃, ZnCl₂, FeBr₃, FeCl₃.

6. Procédé selon la revendication 5, **caractérisé en ce qu'**on utilise comme catalyseur de Friedel-Crafts AlCl₃ ou AlBr₃.

7. Procédé selon l'une quelconque des revendications 1 à 6, **caractérisé en ce qu'**on utilise le catalyseur de Friedel-Crafts en une quantité de 1 à 200 % en moles, en particulier comprise entre 33 et 110 % en moles, par rapport à la quantité molaire du phénylpropanol p-substitué qui est utilisé.

8. Procédé selon l'une quelconque des revendications 1 à 7, **caractérisé en ce qu'**on effectue l'isomérisation à une température comprise entre 0 et 100 °C.

9. Procédé selon l'une quelconque des revendications 1 à 7, **caractérisé en ce qu'**on isomérise du 2-méthyl-3-(4-tert-butylphényl)propanol en 2-méthyl-3-(3-tert-butylphényl)propanol.

10. Procédé selon l'une quelconque des revendications 1 à 7, **caractérisé en ce qu'**on effectue l'isomérisation en partant de 2-méthyl-3-(4-isobutyl-phényl)propanol, 3-(4-tert-butylphényl)propanol, 2-méthyl-3-(4-isopropylphényl)propanol, 3-(4-éthyl-phényl)-2,2-diméthylpropanol ou 3-(4-isopropylphényl)-butanol.

11. Procédé pour la fabrication de substances odorantes de formule (VI) 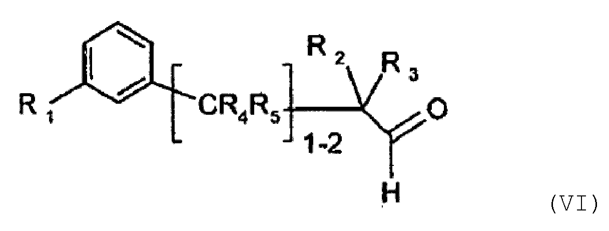 dans laquelle R₁ représente un groupe alkyle en C₁-C₅, et R₂, R₃, R₄ et R₅ représentent, indépendamment les uns des autres, un atome d'hydrogène ou le groupe méthyle, **caractérisé en ce qu'**on prépare un phénylalcanol m-substitué de formule (I) 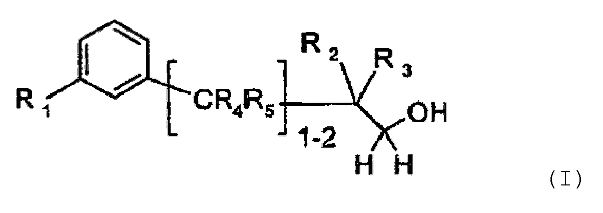 conformément au procédé selon la revendication 1 et ensuite on convertit le phénylalcanol m-substitué de formule (I) obtenu, par oxydation ou déshydrogénation, en le phénylalcanal m-substitué de formule (VI).
